# EUROPEAN PATENT APPLICATION

(11) **EP 1 829 958 A1**
(43) Date of publication of application: **05.09.2007**
(21) Application number: 04817136.7
(22) Date of filing: 30.11.2004
(51) Int. Cl.: C12M 1/34, C12M 1/26, C12Q 1/66, C12Q 1/24

(54) **MEASURING KIT FOR MICROBE IN LIQUID SAMPLE, AND RELEVANT MEASURING METHOD AND MEASURING APPARATUS**

(71) Applicant: DML CO., LTD., Ishikawa 921-8021 (JP)
(72) Inventor: TANAKA, Kojiro, Ishikawa 920-8021 (JP)
(74) Representative: TBK-Patent
(86) International application number: PCT/JP2004/017747
(87) International publication number: WO 2006/059359

(57) **Abstract**

To be able to easily and rapidly remove free ATP, extract ATP from trapped microorganisms and measure extracted ATP, that loses less microorganisms in a sample, that does not require skill and that can measure the microorganisms in the sample stably and high-sensitively.

A flocculant 3 is kept sucked beforehand in a first syringe 2. Then, a liquid sample LS is sucked and agitated. Then, a first filter case 5 and a second filter case 6 are attached immediately to a leading end. Then, this mixture liquid 15 is filtered. Then, only the second filter case 6 is detached and a washing liquid 8 is kept sucked by a second syringe 7 so as to wash the second filter case 6. Next, a bacteriolytic agent 9 is filled in the second filter case 6 and reacted for about 30 seconds. Then, a reacted liquid 16 is pushed out to a measuring tube 10. Then, a luminous reagent (11a+11b) that is prepared beforehand is added. Thereafter, an adapter 12 is attached.

Then, it is agitated lightly Then, a luminous quantity is measured by a luminometer at once.

## Description

### TECHNICAL FIELD

The present invention relates to a measuring kit, a measuring method and a measuring apparatus that is able to promptly and easily and high-sensitively measure microorganism in a liquid sample such as food, water or cosmetics containing much free ATP (somatic cell ATP), e.g. dry food, milk, dairy product or the like, that is regarded as an object in which microorganism is difficult to be measured by a normal ATP method.

### BACKGROUND ART

A standard agar plate method is generally used for measuring microorganism in food or the like. However, this method requires much time period such as 24 to 48 hours to determine it. Therefore, if there occurs microbial contamination, rapid countermeasure is impossible. As a more easy-to-use and rapid detection method of microorganisms, there is known a bio-luminescence method (ATP method) that measures a quantity of adenosine triphosphate (ATP) in microorgansms by measuring a luminous quantity by use of a luminometer, thereby calculating a number of the microorganisms. However, according to this method, in case a number of microorganisms is few in a fluid specimen or is not more than 10³ to 10⁴/ml, such number falls below measurable limits of the luminometer, so that detection is impossible.

On the other hand, most foods contain free ATP that is not derived from microorganisms. In most case, a concentration of such free ATP is incomparably high as compared with a concentration of ATP derived from microorganisms. Therefore, there is a problem that the ATP in the microorganisms cannot be correctly measured without removing interruption of them in advance. In order to solve these problems, there is proposed a method of applying the ATP method after degrading the free ATP not derived from microorganisms in advance by use of filtering the fluid specimen or using apyrase as an ATP degrading enzyme or the like.

However, it is necessary to heighten a concentration of the ATP degrading enzyme in a measured sample in order to degrading the free ATP in a short time. On the other hand, there arises a problem that too high concentration of the degrading enzyme restrains luminescence of the ATP of microorganism origin that is extracted in a next step, thereby lessening a measurement concentration significantly.

There is proposed a method of filtering the fluid specimen so as to trap microorganisms contained therein on a filter, flush an ATP degrading reagent attached to the filter and the microorganisms on the filter and extract the ATP in the microorganisms. However, this method is restricted to food samples (alcoholic beverages, cooking sauce or the like) that are easy to filter. It is not applicable to samples having high turbidity containing a large amount of protein, fat or fat-free solid content such as commercially available milk or dairy products, since their filtering is difficult.

There is proposed a method of treating by diluting samples of such dairy products that are difficult to filter, thereby measuring a microbial ATP in a diluted liquid. This method obtain 0.1ml of the sample, adds and mixes 0.1ml of processing solvent, 0.7ml of diluted solution and 0.1ml of ATP eliminating agent to it, fractionates 0.1ml from 1.0ml of such mixture in a measuring tube and leaves it for 30 minutes, then adding 0.1ml of luminous reagent and measures a luminous quantity by a luminometer. It measures a microbial ATP in a very slight amount of the sample such as 0.01ml, so that the luminous quantity is very small. Accordingly, a correct measurement result is not obtained unless a reagent blank value is always a low value of not more than 50 (RLU) in the luminous quantity. Therefore, there are many problems that an examination or the like is necessary whether the used processing solvent, the diluted solvent, the extracted agent and the high-sensitive luminous reagent are contaminated or not.

Then, the present applicant discloses a method that mixes a food sample with a phosphoric acid flocculant and a filtering accelerating agent such as an ethylenediamine tetraacetic acid salt or the like, carries out a natural filtering by gravity, then filters a filtered liquid by a membrane filter under reduced pressure so as to trap and condense microorganisms in the sample on a filtration film, rinses out and removes free ATP that are not derived from the microorganisms on the filtration film by a washing liquid, then adding ATP extracting reagent (bacteriolytic agent) in the microorganisms, adding a luminous reagent in an extracted liquid and measures a luminous quantity generated, thereby measuring the microorganisms in the food, in a first patent publication. According to this method, the microorganisms can be measured very easily, rapidly and high-sensitively as compared with a conventional method.
First Patent Publication: Laid Open Patent Publication No. 2003-284589

### DISCLOSURE OF THE INVENTION

### PROBLEMS THAT THE INVENITON IS TO SOLVE

However, in the method disclosed in the first patent publication, a phosphoric acid flocculant is used. Consequently, an unskilled person may flocculate protein and microorganisms without distinction at the time of flocculating reaction so as to lose the microorganisms in the sample. Moreover, since the phosphoric acid flocculant coats the microorganisms at the time of extracting the ATP, the bacteriolytic agent is hard to work. Then, it is indispensable to wash with a large quantity of washing liquid. As a result, there is a fear that it becomes a factor of instability of a test result.

In view of the above, it is an object of the present invention to provide a measuring kit, a measuring method and a measuring apparatus of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

### MEANS TO SOLVE THE PROBLEMS

A measuring kit of microorganisms in a liquid sample according to an invention of claim 1 comprises a first syringe for collecting a liquid sample, a flocculant for flocculating protein in the liquid sample in the first syringe, a first filter case, attachable to the first syringe, for housing a first filter that traps the flocculated protein and somatic cells and that transports free ATP (adenosine triphosphate) and microorganisms, a second filter case, attachable to the first filter case, for housing a second filter that traps the microorganisms and that transports the free ATP, a second syringe that can attach the second filter case to its leading end, a washing liquid for washing the second filter, a bacteriolytic agent for dissolving the microorganisms trapped on the second filter so as to dissolve out ATP, a measuring tube for gathering the dissolved ATP together with the bacteriolytic agent, and a luminous reagent for making the dissolved ATP glow.

A measuring kit of microorganisms in a liquid sample according to an invention of claim 2, in a constitution of claim 1, further comprises a luminometer for measuring a luminous quantity and an adapter attached to the measuring tube so as to make a leading end of the measuring tube reach a luminosity measuring portion of the luminometer.

A measuring kit of microorganisms in a liquid sample according to an invention of claim 3, in a constitution of claim 1 or claim 2, further comprises a filtering accelerating agent for making filtering perform in a short time or uses one mixing beforehand the filtering accelerating agent as the flocculant.

In a measuring kit of microorganisms in a liquid sample according to an invention of claim 4, in a constitution of one of claim 1 to claim 3, the first filter and the second filter are assembled integrally in the first filter case and the second filter case and made disposable.

A measuring method of microorganisms in a liquid sample according to an invention of claim 5 comprises a step for mixing a liquid sample with a flocculant that flocculates protein in the liquid sample, a step for filtering under pressure or under reduced pressure by a first filter that traps the flocculated protein and somatic cells and that transports free ATP (adenosine triphosphate) and microorganisms, a step for filtering under pressure or under reduced pressure a filtered liquid by a second filter that has a pore diameter smaller than the first filter and that traps the microorganisms while transporting the free ATP so as to trap and condense the microorganisms in the liquid sample on a filtration film of the second filter, and a step for adding a bacteriolytic agent in the microorganisms, adding a luminous reagent in an extracted liquid and measuring a luminous quantity generated.

A measuring method of microorganisms in a liquid sample according to an invention of claim 6, in a constitution of claim 5, adds a step for adding a filtering accelerating agent for making filtering in a short time after the step for mixing the liquid sample with the flocculant that flocculates the protein in the liquid sample or simultaneously with the same step.

A measuring apparatus of microorganisms in a liquid sample according to an invention of claim 7 mixes a liquid sample with a flocculant that flocculates protein in the liquid sample, filters under pressure or under reduced pressure by a first filter that traps the flocculated protein and somatic cells and that transports free ATP (adenosine triphosphate) and microorganisms, filters under pressure or under reduced pressure a filtered liquid by a second filter that has a pore diameter smaller than the first filter and that traps the microorganisms while transporting the free ATP so as to trap and condense the microorganisms in the liquid sample on a filtration film of the second filter, adds a bacteriolytic agent in the microorganisms, adds a luminous reagent in an extracted liquid and measures a luminous quantity generated.

A measuring method of microorganisms in a liquid sample according to an invention of claim 8, in a constitution of claim 7, adds a filtering accelerating agent for making filtering in a short time after mixing the liquid sample with the flocculant that flocculates the protein in the liquid sample or simultaneously therewith.

A measuring kit of microorganisms in a liquid sample or a measuring method of microorganisms in a liquid sample or a measuring apparatus of microorganisms in a liquid sample according to an invention of claim 9, in a constitution of one of claim 1 to claim 4 or claim 5 or claim 6 or claim 7 or claim 8, uses an aliphatic alcohol such as an ethanol, a carboxylic acid such as a benzoic acid or a salicylic acid, a chitosan or a chitosan oligosaccharide.

A measuring kit or a measuring method or a measuring apparatus of microorganisms in a liquid sample according to an invention of claim 10, in a constitution of one of claim 1 to claim 9, uses a filtering material of a pore diameter of about 1µm to about 10µm as the first filter.

In a measuring kit or a measuring method or a measuring apparatus of microorganisms in a liquid sample according to an invention of claim 11, in a constitution of one of claim 1 to claim 10, the second filter is a porous polymer membrane having pores of a pore diameter of about 0.1µm to about 0.5µm.

In a measuring kit or a measuring method or a measuring apparatus of microorganisms in a liquid sample according to an invention of claim 12, in a constitution of claim 11, the porous polymer membrane is made of one polymer among a polytetrafluoroehylene, a polyvinylidene difluoride, a polycarbonate, a cellulose acetate, a hydrophilic polypropylene, a nylon, a hydrophilic polyether sulfonate and hydrophilic borosilicate glass fibers.

In a measuring kit or a measuring method or a measuring apparatus of microorganisms in a liquid sample according to an invention of claim 13, in a constitution of one of claim 1 to claim 12, the bacteriolytic agent is a sterile distilled water containing a dimethylsulfoxide.

In a measuring kit or a measuring method or a measuring apparatus of microorganisms in a liquid sample according to an invention of claim 14, in a constitution of claim 13, the bacteriolytic agent is a sterile distilled water containing about 15% by content to about 20% by content of a dimethylsulfoxide.

A measuring kit or a measuring method or a measuring apparatus of microorganisms in a liquid sample according to an invention of claim 15, in a constitution of one of claim 3 to claim 14, uses an alkali metal salt of an ethylenediaminetetraacetic acid, an alkali metal salt of a trans-1, 2-cyclohexanediaminetetraacetic acid, an alkali metal salt of a glycol ether diaminetetraacetic acid, an alkali metal salt of a diethylenetriamine pentaacetic acid, or an alkali metal salt of a nitrilotriacetic acid as the filtering accelerating agent.

A measuring kit or a measuring method or a measuring apparatus of microorganisms in a liquid sample according to an invention of claim 16, in a constitution of one of claim 5 to claim 15, adds a sterile distilled water, in case of a solid sample or a sample of high viscosity, before mixing it with the flocculant and then homogenizes it into the liquid sample.

### EFFECTS OF THE INVENTION

The measuring kit of the microorganisms in the liquid sample according to the invention of claim 1 comprises the first syringe for collecting the liquid sample, the flocculant for flocculating the protein in the liquid sample in the first syringe, the first filter case, attachable to a leading end of the first syringe, for housing the first filter that traps the flocculated protein and the somatic cells and that transports the free ATP and the microorganisms, the second filter case, attachable to a leading end of the first filter case, for housing the second filter that traps the microorganisms and that transports the free ATP, the second syringe that can attach the second filter case to its leading end, the washing liquid for washing the second filter, the bacteriolytic agent for dissolving the microorganisms trapped on the second filter so as to dissolve out the ATP, the measuring tube for gathering the dissolved ATP together with the bacteriolytic agent, and the luminous reagent for making the dissolved ATP glow.

With this, the flocculated protein and the somatic cells (containing the ATP inside) are trapped by the first filter by pushing out the liquid sample from the first syringe after flocculating the protein in the liquid sample in the first syringe by the flocculant in a state that the first filter case is attached to the leading end of the first syringe and the second filter case is attached to the leading end thereof. The free ATP is transmitted through both of the first filter and the second filter and discharged from the leading end of the second filter case, so that only the microorganisms are trapped on the second filter. Then, only the second filter case is detached and attached to a leading end of a new first syringe or a new second syringe so as to wash the second filter sufficiently by ejecting the washing liquid. Thereafter, the bacteriolytic agent is sucked in the second syringe, the second filter case is attached to the leading end and the bacteriolytic agent is introduced to react so as to dissolve the microorganisms trapped on the second filter and dissolve out the ATP inside it.

The sample liquid thus extracting only the ATP derived from the microorganisms is collected in the measuring tube and added with the luminous reagent. Then, the luminous quantity is measured. Thereby, it is possible to measure the microorganisms in the liquid sample in a very short time and with accuracy. Moreover, since loss of the microorganisms is less, it is possible to obtain a test result with very high reliability. Since these necessary instruments and reagent are in a kit, it is possible to easily measure a quantity of microorganisms in a short time even at a farm or a factory, a field of food production or a field of cosmetic development or the like by carrying them in a special case.

Thus, it becomes a measuring kit of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

The measuring kit of the microorganisms in the liquid sample according to the invention of claim 2, in the constitution of claim 1, further comprises the luminometer for measuring the luminous quantity and the adapter attached to the measuring tube so as to make the leading end of the measuring tube reach the luminosity measuring portion of the luminometer.

The luminometer is a measuring instrument with which anyone can easily and correctly measure the luminous quantity anywhere without skill. Then, an adapter attachable to the measuring tube is required in order to make the leading end of the measuring tube reach the luminosity measuring portion of the luminometer. It is possible to measure the quantity of the microorganisms in the liquid sample in a very short time and with accuracy by adding them to the constitution of claim 1. Moreover, since the loss of the microorganisms is less, it is possible to obtain a test result of very high reliability Since these necessary instruments and reagent are in a kit, it is possible to easily measure a quantity of microorganisms in a short time even at a farm or a factory, a field of food production or a field of cosmetic development or the like by carrying them in a special case.

Thus, it becomes a measuring kit of microorganisms in a liquid sample that can easily and rapidly remove free ATP, extract ATP from trapped microorganisms and measure extracted ATP, that does not require skill, that enables examination in a filed of production of foods or a field of development of cosmetics or the like and that can measure the microorganisms in the sample stably and high-sensitively.

The measuring kit of the microorganisms in the liquid sample according to the invention of claim 3, in the constitution of claim 1 or claim 2, further comprises the filtering accelerating agent for making filtering perform in a short time or uses one mixing beforehand the filtering accelerating agent as the flocculant. As the filtering accelerating agent, a sodium salt or the like such as an ethylenediamine tetraacetic acid (EDTA) can be used. If the filtering accelerating agent is added after flocculating the protein in the liquid sample by the flocculant in the first syringe or simultaneously with the flocculant, even a beginner can smoothly carry out a filtering processing by the first filter case and the second filter case in a short time so as to measure the quantity of the microorganisms in the liquid sample in a very short time and with accuracy. Moreover, since the loss of the microorganisms is less, it is possible to obtain a test result of very high reliability. Since these necessary instruments and reagent are in a kit, it is possible to easily measure a quantity of microorganisms in a short time even at a farm or a factory, a field of food production or a field of cosmetic development or the like by carrying them in a special case.

Thus, it becomes a measuring kit of microorganisms in a liquid sample that can easily and rapidly remove free ATP, extract ATP from trapped microorganisms and measure extracted ATP, that does not require skill, that enables examination in a filed of production of foods or a field of development of cosmetics or the like and that can measure the microorganisms in the sample stably and high-sensitively.

In the measuring kit of the microorganisms in the liquid sample according to the invention of claim 4, the first filter and the second filter are assembled integrally in the first filter case and the second filter case and made disposable. Thereby, it is possible to save labor of setting up the filter on the filter case or taking it out from the filter case, thereby being able to perform measurement more rapidly. Moreover, it is possible to improve measuring accuracy to enable a measurement with high reliability by making them disposable so as to be used just one time.

Thus, it becomes a measuring kit of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

The measuring method of the microorganisms in the liquid sample according to the invention of claim 5 comprises the step for mixing the liquid sample with the flocculant that flocculates the protein in the liquid sample, the step for filtering under pressure or under reduced pressure by the first filter that traps the flocculated protein and the somatic cells and that transports the free ATP and the microorganisms, the step for filtering under pressure or under reduced pressure the filtered liquid by the second filter that has the pore diameter smaller than the first filter and that traps the microorganisms while transporting the free ATP so as to trap and condense the microorganisms in the liquid sample on a filtration film of the second filter, and the step for adding the bacteriolytic agent in the microorganisms, adding the luminous reagent in the extracted liquid and measuring the luminous quantity generated.

Thereby, the flocculated protein and the somatic cells (containing the ATP inside) are trapped on the first filter by filtering by the first filter under pressure or under reduced pressure after the protein in the liquid sample is flocculated by the flocculant and filtering the filtered liquid by the second filter under pressure or under reduced pressure. The free ATP is transmitted through both of the first filter and the second filter and discharged, so that only the microorganisms are trapped on the second filter. Then, the bacteriolytic agent is added to dissolve the microorganisms trapped on the second filter and dissolve out the ATP inside it. Thereafter, the luminous reagent is added so as to measure the luminous quantity. Thereby, it is possible to measure the microorganisms in the liquid sample in a very short time and with accuracy. Moreover, since loss of the microorganisms is less, it is possible to obtain a test result with very high reliability.

In case it is presumable that the free ATP that is not derived from the microorganisms or other luminous substances are attached to the second filter to such a degree as influences the measurement, a step may be added to wash the second filter with a small amount of washing liquid after filtering under pressure or under reduced pressure if needed.

Thus, it becomes a measuring method of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

The measuring method of the microorganisms in the liquid sample according to the invention of claim 6 adds the step for adding the filtering accelerating agent for making filtering in a short time after the step for mixing the liquid sample with the flocculant that flocculates the protein in the liquid sample or simultaneously therewith. That is, it is possible to use a flocculant that added the filtering accelerating agent beforehand. Thus, the filtering under pressure or under reduced pressure in the first filter or the filtering under pressure or under reduced pressure in the second filter smoothly progresses in a short time by adding the filtering accelerating agent after or simultaneously with flocculating the protein in the liquid sample by the flocculant. Consequently, it becomes a measuring method of microorganisms in a liquid sample that can measure the microorganisms in the sample stably and high-sensitively in a shorter time.

The measuring apparatus of the microorganisms in the liquid sample according to the invention of claim 7 mixes the liquid sample with the flocculant that flocculates the protein in the liquid sample, filters under pressure or under reduced pressure by the first filter that traps the flocculated protein and the somatic cells and that transports the free ATP and the microorganisms, filters under pressure or under reduced pressure the filtered liquid by the second filter that has the pore diameter smaller than the first filter and that traps the microorganisms while transporting the free ATP so as to trap and condense the microorganisms in the liquid sample on the filtration film of the second filter, adds the bacteriolytic agent in the microorganisms, adds the luminous reagent in the extracted liquid and measures the luminous quantity generated.

Thereby, the flocculated protein and the somatic cells (containing the ATP inside) are trapped on the first filter by filtering by the first filter under pressure or under reduced pressure after the protein in the liquid sample is flocculated by the flocculant and filtering the filtered liquid by the second filter under pressure or under reduced pressure. The free ATP is transmitted through both of the first filter and the second filter and discharged, so that only the microorganisms are trapped on the second filter. Then, the bacteriolytic agent is added to dissolve the microorganisms trapped on the second filter and dissolve out the ATP inside it. Thereafter, the luminous reagent is added so as to measure the luminous quantity. Thereby, it is possible to measure the microorganisms in the liquid sample in a very short time and with accuracy. Moreover, since loss of the microorganisms is less, it is possible to obtain a test result with very high reliability.

In case it is presumable that the free ATP that is not derived from the microorganisms or other luminous substances are attached to the second filter to such a degree as influences the measurement, the second filter may be washed with a small amount of washing liquid after filtering under pressure or under reduced pressure if needed.

Thus, it becomes a measuring apparatus of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

The measuring method of the microorganisms in the liquid sample according to the invention of claim 8 adds the filtering accelerating agent for making filtering in a short time after mixing the liquid sample with the flocculant that flocculates the protein in the liquid sample or simultaneously therewith. That is, it is possible to use a flocculant that added the filtering accelerating agent beforehand Thus, the filtering under pressure or under reduced pressure in the first filter or the filtering under pressure or under reduced pressure in the second filter smoothly progresses in a short time by adding the filtering accelerating agent after or simultaneously with flocculating the protein in the liquid sample by the flocculant. Consequently, it becomes a measuring apparatus of microorganisms in a liquid sample that can measure the microorganisms in the sample stably and high-sensitively in a shorter time.

The measuring kit or the measuring method or the measuring apparatus of the microorganisms in the liquid sample according to the invention of claim 9 uses the aliphatic alcohol such as the ethanol, the carboxylic acid such as the benzoic acid or the salicylic acid, the chitosan or the chitosan oligosaccharide. These flocculants selectively coagulate only the protein in the liquid sample and never take in the microorganisms together. Therefore, it is possible to curb and lessen the loss of the microorganisms in measurement and to measure the quantity of the microorganisms in the liquid sample in a very short time and with accuracy.

Thus, it becomes a measuring kit or a measuring method or a measuring apparatus of microorganisms in a liquid sample that can easily and rapidly remove free ATP, extract ATP from trapped microorganisms and measure extracted ATP, that does not need washing by a washing liquid in any way or does not need washing by a large quantity of washing liquid, that loses less microorganisms in a sample, that does not require skill, that enables examination in a filed of production of foods or a field of development of cosmetics or the like and that can measure the microorganisms in the sample stably and high-sensitively.

The measuring kit or the measuring method or the measuring apparatus of the microorganisms in the liquid sample according to the invention of claim 10 uses the filtering material of the pore diameter of about 1µm to about 10µm as the first filter. Thus, it is possible to surely trap the coagulated protein and the somatic cells, while surely transporting the microorganisms and the free ATP, so as to measure the quantity of the microorganisms with high precision.

In the measuring kit or the measuring method or the measuring apparatus of the microorganisms in the liquid sample according to the invention of claim 11, the second filter is the porous polymer membrane having the pores of the pore diameter of about 0.1µm to about 0.5µm. Thus, it is possible to surely trap only the microorganisms, while surely transporting the free ATP, so as to measure the quantity of the microorganisms with high precision.

In the measuring kit or the measuring method or the measuring apparatus of the microorganisms in the liquid sample according to the invention of claim 12, the porous polymer membrane is made of one polymer among the polytetrafluoroehylene, the polyvinylidene difluoride, the polycarbonate, the cellulose acetate, the hydrophilic polypropylene, the nylon, the hydrophilic polyether sulfonate and the hydrophilic borosilicate glass fibers.

The porous polymer membrane made of these polymers can surely trap only the microorganisms, while surely transporting the free ATP, so as to measure the quantity of the microorganisms with high precision.

In the measuring kit or the measuring method or the measuring apparatus of the microorganisms in the liquid sample according to the invention of claim 13, the bacteriolytic agent is the sterile distilled water containing the dimethylsulfoxide (referred to as "DMSO" hereafter).

As a result of keen experimental work of the present inventor, it is found that, if a surfactant compound is used as the bacteriolytic agent, it foams when passing the second filter and does not go well and that the sterile distilled water of DMSO can be used as the bacteriolytic agent without any problem in case of using it of all others. The inventor has completed the present invention on the basis of this expertise.

Thus, it becomes a measuring kit or a measuring method or a measuring apparatus of microorganisms in a liquid sample that can easily and rapidly extract ATP from trapped microorganisms and measure extracted ATP, that does not require skill, that enables examination in a filed of production of foods or a field of development of cosmetics or the like and that can measure the microorganisms in the sample stably and high-sensitively

In the measuring kit or the measuring method or the measuring apparatus of the microorganisms in the liquid sample according to the invention of claim 14, the bacteriolytic agent is the sterile distilled water containing about 15% by content to about 20% by content of the dimethylsulfoxide.

Though the DMSO is excellent as the bacteriolytic agent, the bacteriolytic function (ATP extracting function) drastically deteriorates if a content of the DMSO in the sterile distilled water is too small, while the luminous quantity declines if the content is too large, thereby disturbing the measurement. Then, as a result of keen experimental work of the present inventor, it is found that, if the sterile distilled water containing about 15% by content to about 20% by content of the DMSO as the bacteriolytic agent, a sufficient bacteriolytic function is obtained without diminishing the luminous quantity. The inventor has completed the present invention on the basis of this expertise.

Thus, it becomes a measuring kit or a measuring method or a measuring apparatus of microorganisms in a liquid sample that can easily and rapidly extract ATP from trapped microorganisms and measure extracted ATP, that does not require skill, that enables examination in a filed of production of foods or a field of development of cosmetics or the like and that can measure the microorganisms in the sample stably and high-sensitively.

The measuring kit or the measuring method or the measuring apparatus of the microorganisms in the liquid sample according to the invention of claim 15 uses the alkali metal salt of the ethylenediaminetetraacetic acid (EDTA), the alkali metal salt of the trans-1, 2-cyclohexanediaminetetraacetic acid (CyDTA), the alkali metal salt of the glycol ether diaminetetraacetic acid (GEDTA), the alkali metal salt of the diethylenetriamine pentaacetic acid (DTPA), or the alkali metal salt of the nitrilotriacetic acid (NTA) as the filtering accelerating agent. As the alkali metal, a sodium or a potassium is preferable.

These filtering accelerating agents are compounds used generally as chelating agents. However, if they are used together with the flocculant, they form a chelate compound (water-soluble) with a mineral contained in the liquid sample like a milk or the like, e.g. with a calcium, thereby facilitating the filtering. An added amount of the above-mentioned filtering accelerating agent varies depending on a kind of a used filtering accelerating agent or a liquid sample and cannot be uniquely determined. Still, 1mg to 20mg per 1ml of the liquid sample is generally enough, and 1mg to 20mg per 1ml of a commercially available milk is generally enough in case of a sodium salt of the EDTA..

The measuring kit or the measuring method or the measuring apparatus of the microorganisms in the liquid sample according to then invention of claim 16 adds the sterile distilled water, in case of the solid sample or the sample of high viscosity, before mixing it with the flocculant and then homogenizes it into the liquid sample.

The measuring method of the microorganisms in the liquid sample according to the present invention is also applicable to a measurement of an amount of microorganisms not only in the sample that is liquid from the beginning such as the milk or the like but also in the solid sample such as meats or the like. In that regard, it is homogenized to be the liquid sample after adding the sterile distilled water.

Thus, it becomes a measuring method or a measuring apparatus of microorganisms in a liquid sample that is also applicable to the measurement of the amount of the microorganisms in the solid sample.

### BRIEF DESCRIPTION OF THE DRAWINGS

[Fig. 1] Fig. 1 is a perspective view showing a measuring kit of microorganisms in a liquid sample according to a first embodiment of the invention.
[Fig. 2] Fig. 2 (a) and (b) is a pattern diagram showing a measurement principle of a measuring method of microorganisms in a liquid sample according to the first embodiment and a second embodiment of the invention.
[Fig. 3] Fig. 3 (a), (b), (c), (d), (e) and (f) is an explanatory drawing showing a sequence of the measuring method of the microorganisms in the liquid sample according to the first embodiment and the second embodiment of the invention.
[Fig. 4] Fig. 4 is a drawing showing a measurement result of a first example in the measuring method of the microorganisms in the liquid sample according to the first embodiment of the invention, while compared with a first comparative example, in case the liquid sample is a commercially available milk.
[Fig. 5] Fig. 5 is a drawing showing a measurement result of a second example in the measuring method of the microorganisms in the liquid sample according to the first embodiment of the invention in case the liquid sample is the commercially available milk.
[Fig. 6] Fig. 6 is a drawing showing a measurement result of the second example in the measuring method of the microorganisms in the liquid sample according to the first embodiment of the invention in case the liquid sample is a freshly drawn raw milk.

### DESCRIPTION OF SYMBOLS

- 1: Measuring kit of microorganisms in a liquid sample
- 2: First syringe
- 3: Flocculant
- 5: First filter case
- 5a: First filter
- 6: Second filter case
- 6a: Second filter
- 7: Second syringe
- 8: Washing liquid
- 9: Bacteriolytic agent
- 10: Measuring tube
- 11: Luminous reagent
- 12: Adapter
- 13: Luminometer
- BC: Somatic cell
- FA: Free ATP
- GRP: Flocculated protein
- LS: Liquid sample
- MO: Microorganism
- PR: Protein

### BEST MODES FOR EMBODYING THE INVENTION

Embodiments of the invention are described hereinafter referring to the drawings.

### FIRST EMBODIMENT

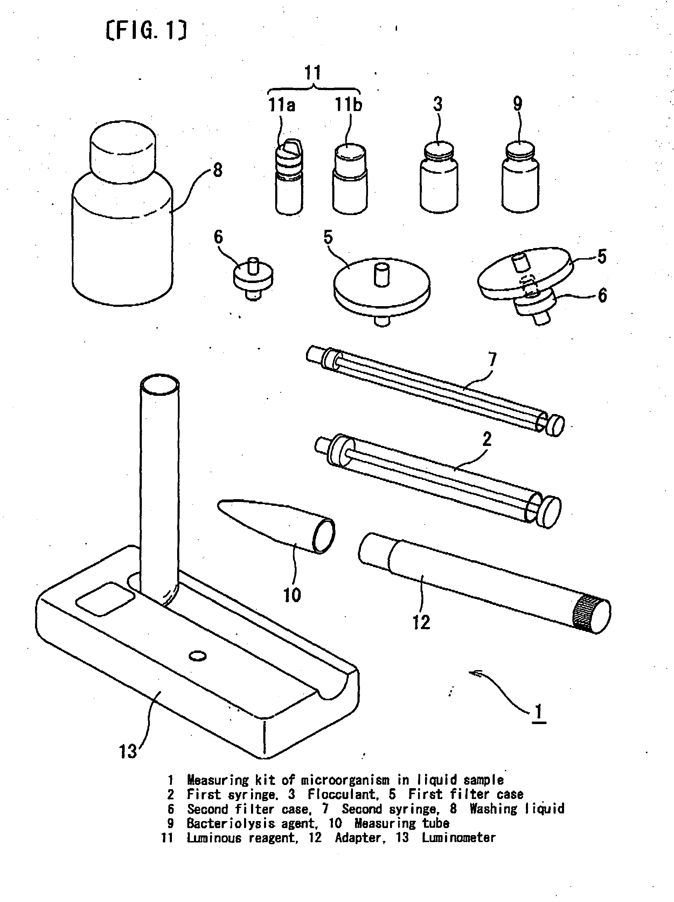

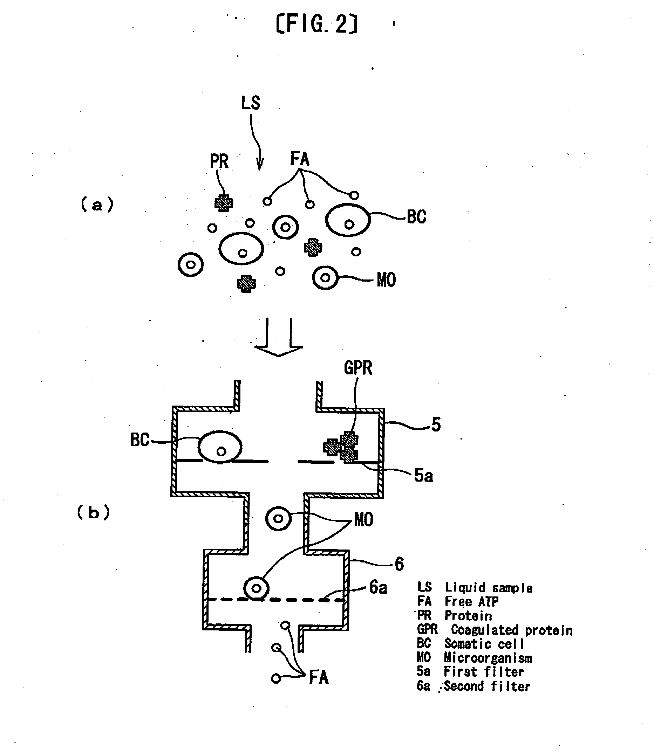

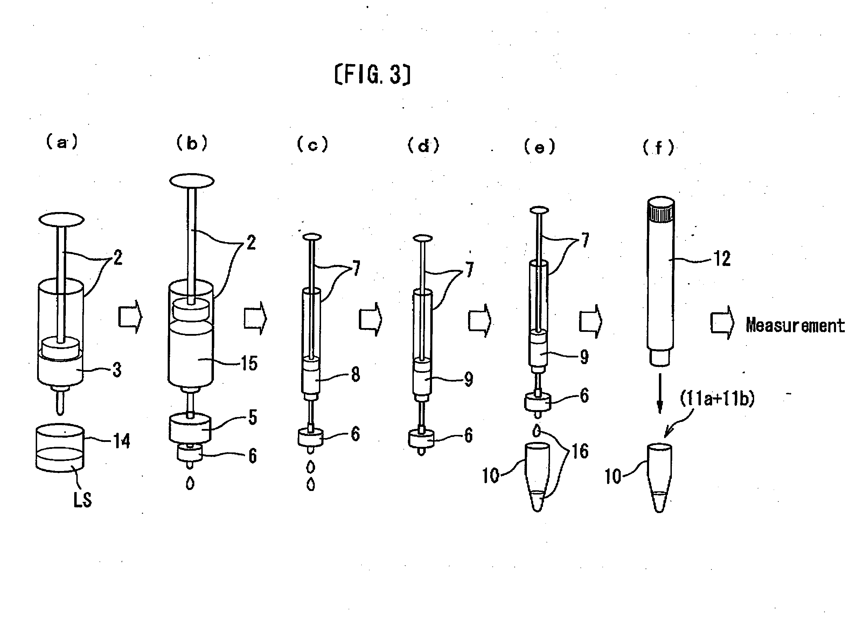

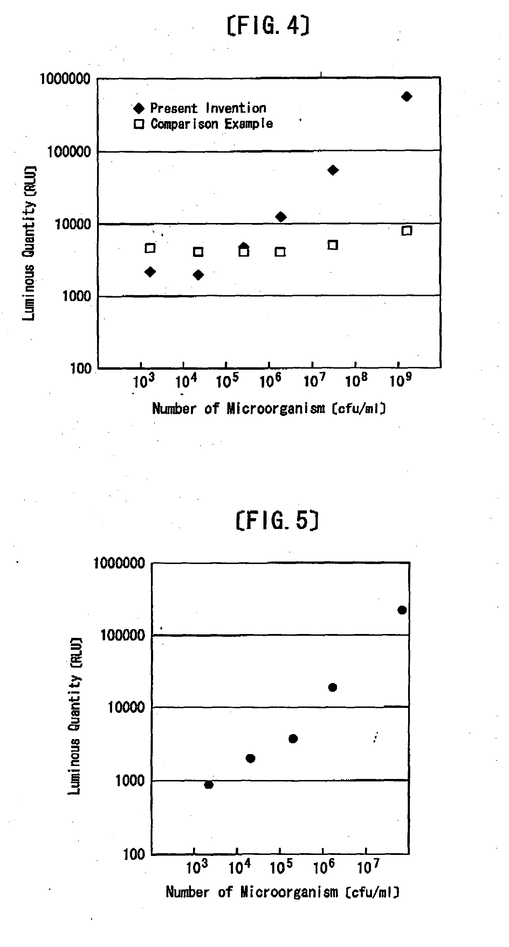

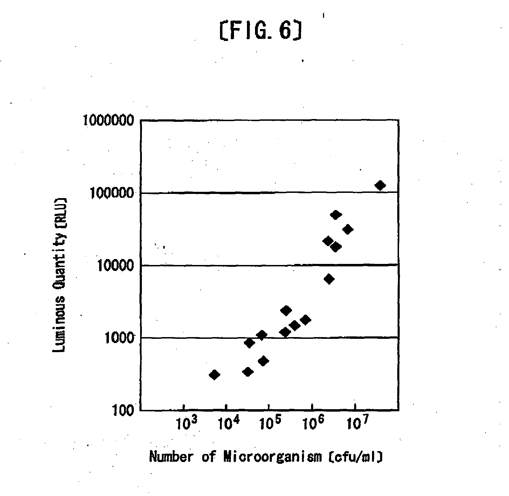

First, a measuring kit, a measuring method and a measuring apparatus of microorganisms in a liquid sample according to a first embodiment of the invention is described referring to Fig. 1 to Fig. 6. Fig. 1 is a perspective view showing a measuring kit of microorganisms in a liquid sample according to the first embodiment of the invention. Fig. 2 (a) and (b) is a pattern diagram showing a measurement principle of a measuring method or a measuring apparatus of microorganisms in a liquid sample according to the first embodiment of the invention. Fig. 3 (a), (b), (c), (d), (e) and (f) is an explanatory drawing showing a sequence .of the measuring method of the microorganisms in the liquid sample according to the first embodiment of the invention. Fig. 4 is a drawing showing a measurement result of a first example in the measuring method of the microorganisms in the liquid sample according to the first embodiment of the invention, while compared with a first comparative example, in case the liquid sample is a commercially available milk. Fig. 5 is a drawing showing a measurement result of a second example in the measuring method of the microorganisms in the liquid sample according to the first embodiment of the invention in case the liquid sample is the commercially available milk. Fig. 6 is a drawing showing a measurement result of the second example in the measuring method of the microorganisms in the liquid sample according to the first embodiment of the invention in case the liquid sample is a freshly drawn raw milk.

In the present invention, the liquid sample is not limited to a specific one. A milk or a variety of dairy products, a soya milk with high protein content or the like will be cited, and cosmetics or water itself can be a sample. A solid sample or a sample having a high viscosity is made into a liquid sample by homogenizing after adding a sterile distilled water.

As shown in Fig. 1, a measuring kit 1 of microorganisms in a liquid sample according to the present first embodiment has a first syringe 2 (3ml content) made of a plastic, a bottled flocculant 3, a first filter case 5 made of a polypropylene housing a first filter, a second filter case 6 made of a polypropylene housing a second filter, a second syringe 7 (1ml content) made of a plastic, a bottled washing liquid 8, a bottled bacteriolytic agent 9, a measuring tube 10 made of a plastic, a luminous reagent 11 composed of a bottled luminous reagent solution 11a and bottled luminous reagent powders 11b, an adapter 12 attached to the measuring tube 10 at the time of measurement, and a luminometer 13 that inserts the measuring tube 10 with the adapter 12 attached in a standing cylindrical portion so as to measure a luminous quantity.

Next described referring to Fig. 2 is a measuring principle of a measuring method of microorganisms in a liquid sample according to the present first embodiment using such measuring kit 1 of the microorganisms in the liquid sample. As shown in Fig. 2(a), free ATP FA, somatic cells BC (containing ATP therein), protein PR and the like are mixed in a liquid sample LS such as a milk or the like in addition to microorganisms MO. Accordingly, unless these substances are removed from the liquid sample LS, it is impossible to measure a mixed amount of the microorganisms MO with accuracy. Then, a flocculant for flocculating only the protein PR is mixed in the liquid sample LS so as to react therewith, thereby making flocculated or coagulated protein GPR

As a flocculant for flocculating or coagulating only the protein PR, there is an aliphatic alcohol such as an ethanol, a carboxylic acid such as a benzoic acid or a salicylic acid, a chitosan, a chitosan oligosaccharide or the like. These flocculants can flocculate the protein PR, but they do not flocculate the microorganisms MO together at that time. Therefore, loss of the microorganisms MO is small.

Thereby, as shown in Fig. 2(b), the liquid sample LS is passed through the connected first filter case 5 and second filter case 6, so that the coagulated protein GPR and the somatic cells BC are trapped and removed at a first filter 5 that traps the coagulated protein GPR and the somatic cells BC and that transports the microorganisms MO. Moreover, the microorganisms MO are trapped at a second filter 6a that traps the microorganisms MO and that transports the free ATP FA. The free ATP FA passes therethrough so as to be discharged and taken off. Then, the ATP in the microorganisms MO are taken out of the second filter 6a. Thereafter, the luminous reagent is put therein so as to measure the luminous quantity. Thus, the amount of the microorganisms MO contained in the initial liquid sample LS is measured with high accuracy.

It is preferable to use a membrane filter of a pore diameter of about 1µm to about 10µm as the first filter 5a. As the membrane filter, for example, a hydrophilic polyether sulfonate (one filter made by two pieces) using a borosilicate glass as a prefilter, a membrane filter of a polyester nonwoven fabric coated with a cellulose acetate, hydrophilic borosilicate glass fibers, a nylon, a polytetrafluoroethylene (PTFE), a polycarbonate, a cellulose acetate, a cellulose mixed ester or the like can be used. The membrane filter has a constant pore diameter and is strong to pressure. Therefore, it is possible to do a stable test by using the membrane filter as the first filter 5a.

The membrane filter as the second filter 6a preferably uses a porous polymer membrane having pores of a pore diameter of about 0.1µm to about 0.5µm or preferably of a pore diameter of about 0.2µm to about 0.45µm. As the porous polymer membrane, a separation membrane made of a polymer of a PTFE, a polyvinylidene difluoride, a polycarbonate, a cellulose acetate, a hydrophilic polypropylene, a nylon, a hydrophilic polyether sulfonate or hydrophilic borosilicate glass fibers or the like can be used.

If filtered by the above-mentioned membrane filter, the microorganisms MO are trapped and condensed on the filtration film 6a. The free ATP FA that is not derived from the microorganisms MO or the luminous substances are sometimes attached to the filtration film 6a and the microorganisms MO. Then, they are washed and removed with the washing liquid if required. As the washing liquid, 60% to 100% by content of a sterile distilled water solution of an ethanol or 1% by content to 8% by content (more preferably 4% by content to 5% by content) of a sterile distilled water solution of a DMSO or the like can be used, for example.

Next described referring to Fig. 3 is a specific sequence of the measuring method of the microorganisms by the measuring kit 1 of the microorganisms in the liquid sample according to the present first embodiment that puts such measuring principle into practice. First, as shown in Fig. 3(a), a fixed amount (2.1ml) of the flocculant 3 is kept sucked beforehand in the first syringe 2 (3ml content). Then, the liquid sample LS is put in a sample cup 14, is sucked by the first syringe 2 by a fixed amount (0.7ml) and is agitated for about five seconds with an air introduced therein, while the leading end of the first syringe 2 being held by fingers. A kind and an added amount of the flocculant 3 varies according to a kind of the liquid sample LS. Still, for example, in case of a commercially available milk, if a flocculant of a carboxylic acid (benzoic acid or the like) is added in a proportion of 2.1ml to 0.7ml of the milk, flocculating reaction progresses rapidly.

Next, as shown in Fig. 3(b), the first filter case 5 and the second filter case 6 coupled with each other are attached to the leading end of the first syringe 2 just after the agitation. Then, a mixture liquid 15 is filtered by pushing a piston of the first syringe 2 slowly. After the filtration, the first syringe 2 is detached once to introduce an air. Then, it is attached again to the first filter case 5 and the second filter case 6 coupled with each other, and an air is fed therein so as to filtrate all the sample in the filter. At this time, as mentioned above, a phenomenon shown in Fig. 2(b) is proceeding.

If the entire amount is filtrated, only the second filter case 6 is detached. Then, as shown in Fig. 3(c), the washing liquid 8 is kept sucked by the second syringe 7. Thereafter, the second filter case 6 is attached to the leading end of the second syringe 7 and the second filter case 6 is washed with the washing liquid 8 by pushing a piston of the second syringe 7. After the filtration, the second syringe 7 is detached once and an air is sucked therein. Then, it is attached again to the second filter case 6 and an air is fed therein. Thus, the washing liquid 8 in the second filter case 6 is completely eliminated. Attention should be paid because a following bacteriolytic process becomes deficient if the washing liquid 8 remains.

Next, as shown in Fig. 3(d), the bacteriolytic agent 9 is kept sucked by the second syringe 7 by a fixed amount (0.2ml). Then, the second filer case 6 is attached and the second syringe 7 is turned upside down so as to feed the bacteriolytic agent 9 in the second filter case 6 from below thereby filling the second filter case 6 with the bacteriolytic agent 9. Thereafter, the second filter case 6 is turned around or given a slight vibration so as to make the bacteriolytic agent 9 spread evenly over the second filter 6a and react for about 30 seconds. After the reaction, as shown in Fig. 3(e), a reaction liquid 16 is pushed out to the measuring tube 10. Then, the reaction liquid 16 remaining in the second filter 6a is also pushed out by the air, thereby gathering it completely in the measuring tube 10.

Next, as shown in Fig. 3(f), a fixed amount (0.1ml) of the luminous reagent 11 prepared by adding the luminous reagent solution 11a to the luminous reagent powders 11b is added. Then, the adapter 12 is attached so as to slightly agitate it. Thereafter, the luminous quantity is measured immediately by the luminometer 13. The luminous reagent is not limited to a specific one. Normally, a luciferase and a luciferin are used.

### (FIRST EXAMPLE)

Next, the present invention is described specifically on the basis of a first example of the measuring method of the microorganisms in the liquid sample according to the first embodiment of the present invention.

A standard culture fluid of the present first example and a comparative example contains 0.5% of a yeast extract, 1.5% of a peptone, 0.5% of a sodium chloride and 0.5% of a potassium hydrogenphosphate. A luminous reagent consists mainly of a luciferin, a luciferase and a magnesium acetate as a main component and is dissolved in a Tricine buffer solution.

20ml of the standard culture fluid was added with a small amount of a raw milk, incubated and centrifuged. Then, the culture fluid was thrown away. Thereafter, precipitated microbes were added with 20ml of a commercially available milk, which was checked to be aseptic, and agitated well. This was diluted with a similar commercially available milk in five levels so as to make them samples.

Used as a filter was one using a first filter (material: membrane filter of a polyester nonwoven fabric coated with a cellulose acetate) of a diameter of 47mm and a pore diameter of 10µm and a membrane filter as a second filter (material: polytetrafluoroethylene) of a diameter of 25mm and a pore diameter of 0.5µm while connecting their respective filter covers with a lure fitting.

3ml of the sample was sucked by use of the syringe of 10ml content. Moreover, 7ml of 99.5% ethanol as the flocculant was directly added. Then, it was mixed hard for about 10 seconds and filtered by the above-mentioned filter. At this time, the protein and the like in the sample were trapped on the first filter. It mainly consists of microorganisms that was filtered and collected on the second filter. The free ATP was discharged as a filtered liquid. Next, the second filter was taken out from the filter cover and repositioned on the measuring tube. Then, it was added with 0.2ml of 20% DMSO solution and left for one minute. Thereafter, it was added with 0.1ml of the luminous reagent so as to measure the luminous quantity (RLU) by the luminometer.

Separately, each of the above-mentioned stepwise diluted samples was cultured by a standard culture method. Then, a number of microorganisms were measured. Fig. 4 shows a relation between the number of the measured microorganisms and a luminous quantity. A horizontal axis shows the number of microbes (cfu/ml), and a vertical axis shows the luminous quantity (RLU). Each of them is a logarithmic axis. It was found from this graph that a good proportionality relation was formed between the luminous quantity (ATP concentration) and the number of microbes if it is not less than 10⁴/ml in accordance with the present first embodiment of the measuring method of the microorganisms in the liquid sample.

### [COMPARATIVE EXAMPLE]

As the comparative example, 0.1ml of each sample was added with the 0.2ml of 20% DMSO solution and left for one minute. Then, 0.1ml of the luminous reagent was added so as to measure a luminous quantity by the luminometer.

A relation between a number of microbes and the luminous quantity (ATP concentration) was illustrated by plotting them with white squares. In this case, no correlation was shown between the number of microbes and the luminous quantity.

As described above, the present first embodiment can easily and rapidly remove free ATP, extract ATP from trapped microorganisms and measure extracted ATP and does not need washing by a washing liquid in any way or does not need washing by a large quantity of washing liquid. Moreover, since loss of the microorganisms is less, it is possible to obtain a test result with very high reliability. Furthermore, the present first embodiment is applicable to a milk with a high turbidity and even to dairy products or dry foods, while being able to measure well the ATP derived from the microorganisms up to a low concentration in such a food as a soya milk that contains a lot of protein and mineral and that is hard to be filtered. In addition, in the present first embodiment, since the membrane filter is used as both of the first filter and second filter, a stable test can be performed, no device or technique is required other than the luminometer and a test is possible in a production field without skill.

### (SECOND EXAMPLE)

Next, the present invention is described specifically on the basis of a second example of the measuring kit and the measuring method of the microorganisms in the liquid sample according to the first embodiment of the present invention referring to Fig. 1 to Fig. 3. In the present second example, measurement of microorganisms was conducted by use of the above-mentioned measuring kit 1 of the microorganisms in the liquid sample according to the present first embodiment.

A standard culture fluid of the present second example contains 0.5% of a yeast extract, 1.5% of a peptone, 0.5% of a sodium chloride and 0.5% of a potassium hydrogenphosphate. A luminous reagent 11 is made by dissolving powders 11b, which consists mainly of a luciferin, a luciferase and a magnesium acetate as a main component, in a Tricine buffer solution 11a.

20ml of the standard culture fluid was added with a small amount of a raw milk, incubated and centrifuged. Then, the culture fluid was thrown away. Thereafter, precipitated microbes were added with 20ml of a commercially available milk, which was checked to be aseptic, and agitated well. This was diluted with a similar commercially available milk in five levels so as to make them samples LS. Moreover, they were added with 20ml of a freshly drawn raw milk obtained in a ranch and agitated well in a similar manner. This was diluted with a similar raw milk in five levels so as to make them samples LS, too.

First, as shown in Fig. 3(a), 2.1ml of benzoic acid as the flocculant was kept sucked beforehand in the first syringe 2 (3ml content). Then, the liquid sample LS was put in the sample cup 14 and sucked with the first syringe 2 by 0.7ml. Moreover, an air is introduced therein and it was agitated for about 5 seconds while holding the leading end of the first syringe 2 by fingers. Next, as shown in Fig. 3(b), the first filter case 5 and the second filter case 6 that were coupled with each other were attached to the leading end of the first syringe 2 just after the agitation. Then, the piston of the first syringe 2 was slowly pushed so as to filter the mixture liquid 15. After the filtration, the first syringe 2 was detached once and an air was introduced therein. Then, it was again attached to the first filter case 5 and the second filter case 6 that were coupled with each other. Thereafter, the sample in the filter was all filtered by feeding an air therein.

If the entire amount was filtered, only the second filter 6 was detached. Then, as shown in Fig. 3(c), the sterile distilled water solution 8 of 60% by content of ethanol as the washing liquid was kept sucked by the second syringe 7. Then, the second filter case 6 was attached to the leading end of the second syringe 7. Thereafter, the piston of the second syringe 7 was pushed so as to wash the second filter case 6 with the washing liquid 8. After the filtration, the second syringe 7 was detached once and an air is introduced therein. Then, it was again attached to the second filter case 6 and an air was introduced therein so as to completely remove the washing liquid 8 in the second filter case 6.

The present second example was described on a example using the ethanol solution of 60% by content as the washing liquid 8. Still, a sterile distilled water solution of 1% by content to 8% by content of DMSO or more preferably a sterile distilled water solution of 4% by content to 5% by content of DMSO or the like can be used as the washing liquid instead thereof.

Next, as shown in Fig. 3(d), 0.2ml of 20% by content of DMSO solution 9 as the bacteriolytic agent was kept sucked by the second syringe 7. Then, the second filter case 6 was attached thereto and the second syringe 7 was turned upside down to feed the bacteriolytic agent 9 in the second filter case 6 from below. Thus, the second filter case 6 is filled with the bacteriolytic agent 9. Then, the second filter case 6 was turned around or given a slight vibration so as to make the bacteriolytic agent 9 uniformly reach the second filter 6a, thereby reacting it for about 30 seconds. Thereafter, as shown in Fig. 3(e), the reacting liquid 16 was pushed to the measuring tube 10, while the reacting liquid 16 remaining in the second filter 6a was pushed out by the air, thereby thoroughly gathering it to the measuring tube 10.

Next, as shown in Fig. 3(f), it was added with 0.1ml of the luminous reagent 11 prepared beforehand and agitated lightly while being attached with the adapter 12. Thereafter, the luminous quantity was measured immediately by the luminometer 13. Separately, each of the aforementioned gradually diluted samples was cultured by the standard culture method. Then, a number of microorganisms were measured. A relation between the measured number of the microorganisms and the luminous quantity is shown in Fig. 5 with respect to the commercially available milk, while being shown in Fig. 6 with respect to the raw milk. The raw milk is not aseptic in contrast to the commercially available milk, so that it already produces luminescence in a state of a blank. In Fig. 6, a value subtracting a luminous quantity of the blank is shown as the luminous quantity. The horizontal axis shows the number of microbes (cfu/ml) and the vertical axis shows the luminous quantity (RLU). Each of them is a logarithmic axis. It was found from this graph that a good proportionality relation was formed between the luminous quantity (ATP concentration) and the number of microbes if it is not less than 10⁴/ml in accordance with the present second example of the measuring method of the microorganisms in the liquid sample.

As described above, the measuring kit and the measuring method of the microorganisms in the liquid sample according to the present second example can easily and rapidly remove free ATP, extract ATP from trapped microorganisms and measure extracted ATP and does not need washing by a washing liquid in any way or does not need washing by a large quantity of washing liquid. Moreover, since loss of the microorganisms is less, it is possible to obtain a test result with very high reliability. Furthermore, the measuring kit and the measuring method of the microorganisms in the liquid sample according to the present second example is applicable to a milk with a high turbidity and even to dairy products or dry foods, while being able to measure well the ATP derived from the microorganisms up to a low concentration in such a food as a soya milk that contains a lot of protein and mineral and that is hard to be filtered. In addition, in the present second example, since both of the first filter and second filter are housed in the cases so as to be disposable, a stable test can be performed, no device or technique is required other than the luminometer and a test is possible in a production field without skill.

### SECOND EMBODIMENT

Next, a measuring kit, a measuring method and a measuring apparatus of microorganisms in a liquid sample according to a second embodiment of the invention is described referring to Fig. 1 to Fig. 3. The measuring kit of the microorganisms in the liquid sample according to the present second embodiment further adds a filtering accelerating agent to the measuring kit 1 of the microorganisms in the liquid sample according to the first embodiment shown in Fig. 1. In the present second embodiment, a sodium salt of an ethylenediaminetetraacetic acid (EDTA) is used as the filtering accelerating agent. Alternatively, it is possible to mix the sodium salt of EDTA beforehand in a fixed proportion in the bottled flocculant 3 of the measuring kit 1 shown in Fig. 1. The sodium salt of EDTA is a compound generally used as a chelating agent. However, if it is used in combination with the flocculant, it forms a chelating compound (water-soluble) with a mineral contained in the liquid sample such as the milk or the like, e.g. a calcium, thereby facilitating the filtration.

Accordingly, a measuring principle of the measuring method of the microorganisms according to the present second embodiment becomes as shown in Fig. 2. The water-soluble chelating compound passes through the second filter 6a together with the free ATP FA so as to be discharged outside a system. Thus, the filtration of the liquid sample is made much easier and a measuring time can be shortened.

Next described referring to Fig. 3 is a specific sequence of the measuring method of the microorganisms by the measuring kit of the microorganisms in the liquid sample according to the present second embodiment that puts such measuring principle into practice. First, as shown in Fig. 3(a), a fixed amount (2.1ml) of a mixed agent of the flocculant 3 and the sodium salt of EDTA as the filtering accelerating agent is kept sucked beforehand in the first syringe 2 (3ml content). Then, the liquid sample LS is put in the sample cup 14, is sucked by the first syringe 2 by a fixed amount (0.7ml) and is agitated for about five seconds with an air introduced therein, while the leading end of the first syringe 2 being held by fingers. A kind and an added amount of the flocculant 3 vary according to a kind of the liquid sample LS. Still, for example, in case of a commercially available milk, if a flocculant of a carboxylic acid (benzoic acid or the like) is added in a proportion of 2.1ml to 0.7ml of the milk, flocculating reaction progresses rapidly.

Alternately, a method can be adopted such that the flocculant 3 alone is kept sucked by a fixed amount (2. lml) and the sodium salt of EDTA as the filtering accelerating agent is added just after the liquid sample LS is sucked and agitated, thereby further agitating it for about 5 seconds while holding the leading end of the first syringe 2. Next, as shown in Fig. 3(b), the first filter case 5 and the second filter case 6 coupled with each other are attached to the leading end of the first syringe 2 just after the agitation. Then, the mixture liquid 15 is filtered by pushing the piston of the first syringe 2 slowly. After the filtration, the first syringe 2 is detached once to introduce an air. Then, it is attached again to the first filter case 5 and the second filter case 6 coupled with each other, and an air is fed therein so as to filtrate all the sample in the filter. At this time, as mentioned above, a phenomenon shown in Fig. 2(b) is proceeding.

If the entire amount is filtrated, only the second filter case 6 is detached. Then, as shown in Fig. 3(c), the washing liquid 8 is kept sucked by the second syringe 7. Thereafter, the second filter case 6 is attached to the leading end of the second syringe 7 and the second filter case 6 is washed with the washing liquid 8 by pushing a piston of the second syringe 7. After the filtration, the second syringe 7 is detached once and an air is sucked therein. Then, it is attached again to the second filter case 6 and an air is fed therein. Thus, the washing liquid 8 in the second filter case 6 is completely eliminated. Attention should be paid because a following bacteriolytic process becomes deficient if the washing liquid 8 remains.

Next, as shown in Fig. 3(d), the bacteriolytic agent 9 is kept sucked by the second syringe 7 by a fixed amount (0.2ml). Then, the second filer case 6 is attached and the second syringe 7 is turned upside down so as to feed the bacteriolytic agent 9 in the second filter case 6 from below thereby filling the second filter case 6 with the bacteriolytic agent 9. Thereafter, the second filter case 6 is turned around or given a slight vibration so as to make the bacteriolytic agent 9 spread evenly over the second filter 6a and react for about 30 seconds. After the reaction, as shown in Fig. 3(e), the reaction liquid 16 is pushed out to the measuring tube 10. Then, the reaction liquid 16 remaining in the second filter 6a is also pushed out by the air, thereby gathering it completely in the measuring tube 10.

Next, as shown in Fig. 3(f), a fixed amount (0.1ml) of the luminous reagent 11 prepared by adding the luminous reagent solution 11a to the luminous reagent powders 11b is added. Then, the adapter 12 is attached so as to slightly agitate it. Thereafter, the luminous quantity is measure immediately by the luminometer 13. The luminous reagent is not limited to a specific one. Normally, a luciferase and a luciferin are used.

The measuring apparatus used for the aforementioned measuring method of the microorganisms in the liquid sample is described on an example of measuring by hands by use of the measuring kit 1 or the measuring kit adding the filtering accelerating agent to the measuring kit 1. However, it is possible to make them an automatic measuring apparatus that hardly uses hands.

The measuring kit or the measuring method of the microorganisms in the liquid sample according to the present invention is not limited to each of the above-mentioned embodiments with respect to the other structures, shapes, materials, dimensions, numbers, connecting relations or the like. The measuring method of the microorganisms in the liquid sample according to the present invention is not limited to each of the above-mentioned embodiments with respect to the other steps.

## Claims

1. A measuring kit of microorganisms in a liquid sample **characterized by** comprising:
a first syringe for collecting a liquid sample;
a flocculant for flocculating protein in the liquid sample in the first syringe;
a first filter case, attachable to the first syringe, for housing a first filter that traps the flocculated protein and somatic cells and that transports free ATP (adenosine triphosphate) and microorganisms;
a second filter case, attachable to the first filter case, for housing a second filter that traps the microorganisms and that transports the free ATP;
a second syringe that can attach the second filter case to its leading end;
a washing liquid for washing the second filter;
a bacteriolytic agent for dissolving the microorganisms trapped on the second filter so as to dissolve out ATP;
a measuring tube for gathering the dissolved ATP together with the bacteriolytic agent; and
a luminous reagent for making the dissolved ATP glow.

2. A measuring kit of microorganisms in a liquid sample according to claim 1, **characterized by** further comprising a luminometer for measuring a luminous quantity and an adapter attached to the measuring tube so as to make a leading end of the measuring tube reach a luminosity measuring portion of the luminometer.

3. A measuring kit of microorganisms in a liquid sample according to claim 1 or claim 2, **characterized by** further comprising a filtering accelerating agent for making filtering perform in a short time or uses one mixing beforehand the filtering accelerating agent as the flocculant.

4. A measuring kit of microorganisms in a liquid sample according to one of claim 1 to claim 3, **characterized in that** the first filter and the second filter are assembled integrally in the first filter case and the second filter case and made disposable.

5. A measuring method of microorganisms in a liquid sample **characterized by** comprising:
a step for mixing a liquid sample with a flocculant that flocculates protein in the liquid sample;
a step for filtering under pressure or under reduced pressure by a first filter that traps the flocculated protein and somatic cells and that transports free ATP (adenosine triphosphate) and microorganisms;
a step for filtering under pressure or under reduced pressure a filtered liquid by a second filter that has a pore diameter smaller than the first filter and that traps the microorganisms while transporting the free ATP so as to trap and condense the microorganisms in the liquid sample on a filtration film of the second filter;
and a step for adding a bacteriolytic agent in the microorganisms, adding a luminous reagent in an extracted liquid and measuring a luminous quantity generated.

6. A measuring method of microorganisms in a liquid sample according to claim 5, **characterized by** adding a step for adding a filtering accelerating agent for making filtering in a short time after the step for mixing the liquid sample with the flocculant that flocculates the protein in the liquid sample or simultaneously with the same step.

7. A measuring apparatus of microorganisms in a liquid sample **characterized by** mixing a liquid sample with a flocculant that flocculates protein in the liquid sample;
filtering under pressure or under reduced pressure by a first filter that traps the flocculated protein and somatic cells and that transports free ATP (adenosine triphosphate) and microorganisms;
filtering under pressure or under reduced pressure a filtered liquid by a second filter that has a pore diameter smaller than the first filter and that traps the microorganisms while transporting the free ATP so as to trap and condense the microorganisms in the liquid sample on a filtration film of the second filter ; and
adding a bacteriolytic agent in the microorganisms, adding a luminous reagent in an extracted liquid, and measuring a luminous quantity generated.

8. A measuring method of microorganisms in a liquid sample according to claim 7, **characterized by** adding a filtering accelerating agent for making filtering in a short time after mixing the liquid sample with the flocculant that flocculates the protein in the liquid sample or simultaneously therewith.

9. A measuring kit of microorganisms in a liquid sample according to one of claim 1 to claim 4 or a measuring method of microorganisms in a liquid sample according to claim 5 or claim 6 or a measuring apparatus of microorganisms in a liquid sample according to claim 7 or claim 8, **characterized by** using an aliphatic alcohol such as an ethanol, a carboxylic acid such as a benzoic acid or a salicylic acid, a chitosan or a chitosan oligosaccharide.

10. A measuring kit or a measuring method or a measuring apparatus of microorganisms in a liquid sample according to one of claim 1 to claim 9, **characterized by** using a filtering material of a pore diameter of about 1µm to about 10µm as the first filter.

11. A measuring kit or a measuring method or a measuring apparatus of microorganisms in a liquid sample according to one of claim 1 to claim 10, **characterized in that** the second filter is a porous polymer membrane having pores of a pore diameter of about 0.1µm to about 0.5µm.

12. A measuring kit or a measuring method or a measuring apparatus of microorganisms in a liquid sample according to claim 11, **characterized in that** the porous polymer membrane is made of one polymer among a polytetrafluoroehylene, a polyvinylidene difluoride, a polycarbonate, a cellulose acetate, a hydrophilic polypropylene, a nylon, a hydrophilic polyether sulfonate and hydrophilic borosilicate glass fibers.

13. A measuring kit or a measuring method or a measuring apparatus of microorganisms in a liquid sample according to one of claim 1 to claim 12, **characterized in that** the bacteriolytic agent is a sterile distilled water containing a dimethylsulfoxide.

14. A measuring kit or a measuring method or a measuring apparatus of microorganisms in a liquid sample according to claim 13, **characterized in that** the bacteriolytic agent is a sterile distilled water containing about 15% by content to about 20% by content of a dimethylsulfoxide.

15. A measuring kit or a measuring method or a measuring apparatus of microorganisms in a liquid sample according to one of claim 3 to claim 14, **characterized by** using an alkali metal salt of an ethylenediaminetetraacetic acid, an alkali metal salt of a trans-1, 2-cyclohexanediaminetetraacetic acid, an alkali metal salt of a glycol ether diaminetetraacetic acid, an alkali metal salt of a diethylenetriamine pentaacetic acid, or an alkali metal salt of a nitrilotriacetic acid as the filtering accelerating agent.

16. A measuring kit or a measuring method or a measuring apparatus of microorganisms in a liquid sample according to one of claim 5 to claim 15, **characterized by** adding a sterile distilled water, in case of a solid sample or a sample of high viscosity, before mixing it with the flocculant and then homogenizes it into the liquid sample.
